Europäisches Patentamt

European Patent Office

Office européen des brevets

(19)

(11) Publication number: **0 480 361 A2**

## EUROPEAN PATENT APPLICATION

(12)

(21) Application number: **91117107.2**

(22) Date of filing: **08.10.91**

(51) Int. Cl.5: **G01N 33/58**, G01N 33/533,
//G01N21/77,G01N33/74,
G01N33/574

(30) Priority: **11.10.90 JP 274808/90**

(43) Date of publication of application:
**15.04.92 Bulletin 92/16**

(84) Designated Contracting States:
**AT BE CH DE DK ES FR GB GR IT LI LU NL SE**

(71) Applicant: **TAKEDA CHEMICAL INDUSTRIES, LTD.**
**1-1, Doshomachi 4-chome**
**Chuo-ku, OSAKA(JP)**

(72) Inventor: **Motsenbocker, Marvin A.**
**302, 32-3, Tsukaguchicho 5-chome**
**Amagasaki, Hyogo 661(JP)**
Inventor: **Ichimori, Yuzo**
**725, Hamaderamotomachi 5-cho**
**Sakai, Osaka 592(JP)**
Inventor: **Kondo, Koichi**
**6-3, Kabutodai 3-chome, Kizu-cho**
**Sohraku-gun, Kyoto 619-02(JP)**

(74) Representative: **von Kreisler, Alek, Dipl.-Chem. et al**
**Patentanwälte Von Kreisler-Selting-Werner,**
**Deichmannhaus am Hauptbahnhof**
**W-5000 Köln 1(DE)**

(54) **Quantitation by chemiluminescence using photosenstive substance.**

(57) Certain metal porphines are used as catalysts to generate chemiluminescence from high pH solution without added oxidizing agent. These catalysts can be conjugated to antibodies to provide immunoassay detection systems of greater convenience and sensitivity.

EP 0 480 361 A2

## FIELD OF THE INVENTION

The present invention relates to novel quantitation by chemiluminescence using a photosensitive substance such as metal porphine. More particularly, the present invention relates generally to the field of quantitation of water borne analytes and specifically with chemiluminescence detection techniques and catalysts employed in diagnostic procedures.

## BACKGROUND OF THE INVENTION

Chemistry based measurement procedures are extremely useful for detecting and quantitating various substances of medical, environmental and industrial importance which appear in liquid medium at very low concentrations. Unless the analyte is itself strongly fluorescent or chemiluminescent it is difficult to detect it directly at low concentration. Thus, the presence of analyte is measured indirectly by use of a signal generating molecule (usually a catalyst) coupled to a binding member in a detection step. Preceding this detection step are other chemical reactions, which usually include binding reaction(s) of antibodies or nucleic acid. The binding member is characterized as having a high binding affinity to another molecule in the assay such as an antibody or analyte. The catalyst used to make a conjugate for detection is most commonly an enzyme. The enzymatic detection reactions are characterized as having specific incubation (pH, salt concentration, temperature, substrate, buffer composition, etc.) requirements to which the enzyme is exposed for a period of time after which the product of the enzyme reaction is assayed. Because of the inherent instability of enzymes, diagnostic assays which incorporate an enzyme catalyst tend to have a shorter storage life and require more specific detection reaction conditions than do assays that do not employ an enzyme catalyst.

On the other hand, the sensitivity of an assay is very important and the detection limit of the detection frequently limits the overall sensitivity of the assay. Chemiluminescence detection systems are preferred because they offer the highest sensitivity. Normally, in chemiluminescence, a chemilumigenic compound (compound that can produce light) is oxidized to make a light photon and the photon is counted by a very sensitive measuring machine. The most popular chemiluminescence systems utilize oxidation of luminol by hydrogen peroxide catalyzed by a heme catalyst or heme containing protein. In a survey of various oxidation systems using various catalysts, microperoxidase with hydrogen peroxide was found to give best detection sensitivity [Schroeder and Yeager, Anal. Chem., 50, 1114-1120 (1978)]. The heme containing protein, horseradish peroxidase, has found to have wide application in the oxidation of luminol by hydrogen peroxide.

All of these techniques require the addition of hydrogen peroxide or other strong oxidizing agent such as urea peroxide or perchlorite at high concentration (0.1 mM-10 mM) to the reaction mixture. However, these agents are unstable and inconvenient to store, use and dispose of. Another problem is that they produce a high background from the oxidation of other substances present in the reaction solution (e.g., protein) and by the presence of other substances such as iron which catalyze background oxidation of the chemilumigenic agent. Thus, a system which does not rely on the addition of a strong oxidizing agent at high concentration to the detection reaction solution is desired.

In recent years, a focus in the research of chemiluminescence systems has been in chemistries that do not require addition of a high energy oxidant. One strategy is to use heat to develop the chemiluminescence detection signal [Hummelen et al., Pure Appl. Chemistry, 59, 639-644 (1987)]. Another strategy has been to develop chemilumigenic enzyme substrates that already contain a high energy but stable dioxetane [Bronstein et. al., J. Biolum. Chemilum., 4, 99-111 (1989)].

Although the stability and state of the art sensitivity of these dioxetane labels is a big improvement to the art, the disadvantages of using an enzyme label remain and the detection reaction usually requires a long time period (10 minutes or more) for best sensitivity.

Although most commercial chemiluminescence systems employ an iron porphyrin protein complex acting with hydrogen peroxide to oxidize luminol, it was shown a long time ago that other compounds besides hydrogen peroxide can directly interact with protein iron porphyrins to produce an active oxidized intermediate compound required for catalysis [George, J. Biol. Chem., 201, 413-426 (1953)]. Also, iron in the +2 state has been successfully detected by luminol chemiluminescence in the absence of an added oxidant [Seitz and Hercules, Anal. Chem., 44, 2143-2149 (1972)]. In this system, however, the detection limit was poor (100 pM) and the iron had to be stabilized against oxidation by oxygen before reaction with oxygen and luminol. Also, the system cannot be applied to the measurement of antibody or DNA because there is no-practical way to couple iron to a protein or nucleic acid and make it available as a catalyst for chemiluminescence detection.

Iron porphyrins complexed in protein (as microperoxidase or horseradish peroxidase) as described above are used as antibody labels and are detected by chemiluminescence but hydrogen peroxide has to be added for the chemiluminescence system to work. The iron in porphine has been successfully replaced by manganese in the determination of hydrogen peroxide [Saito et al., Anal. Sci. 3, 171-174 (1987)] but the iron protein compound, peroxidase, had better activity than did the manganese porphine compound. Manganese porphine has also been used to stimulate catalase and peroxidase activity in the system using hydrogen peroxide but did not show improved activity compared with the natural iron protein compounds (e.g., horseradish peroxidase) [Saito et al., Chem. Pharm. Bull., 34, 2885-2889 (1986); and Chem. Pharm. Bull., 35, 869-872 (1987)]. Thus, although there has been some investigation in the area of metal porphine catalyzed reactions, the prepared compounds were less catalytically active than the natural iron porphyrin compounds and, in each case, hydrogen peroxide was used as a substrate of the reaction.

## OBJECTS OF THE INVENTION

The present invention originated from work with photosensitive dyes in high pH luminol solutions. In that system, light is used by photosensitive dye, for example, to make singlet oxygen which in turn generates chemiluminescence from luminol. The surprising discovery was made that some manganese porphines catalyzed luminol chemiluminescence very efficiently in the absence of light and high energy oxidants. Upon further investigation it was found that metal was required in the porphine ring, manganese worked significantly better than iron and electron withdrawing groups on the porphine phenyl rings improved the non-hydrogen peroxide catalyzed chemiluminescence further. Also, upon further investigation, it was found that this activity could be enhanced more than 100 fold by amphipathic substances as additives such as certain detergents or organic acids that presumably interact with the porphine ring to facilitate reaction of the manganese with oxygen or luminol or both.

The general object of the present invention is to simplify the reaction used for the detection step in diagnostics. This object is achieved by eliminating the need for a high energy compound to generate chemiluminescence from luminol. For example, only sodium hydroxide and luminol in water are required for the chemiluminescence detection of metal porphine. The present invention thus allows greater simplicity compared to prior art methods that require for example, mixing of hydrogen peroxide or other oxidant, or heating or light irradiation to generate a high energy oxidant. In preferred embodiments, a manganese-mesotetra-4-carboxyphenyl porphine labeled antibody for example is used in an assay system and the manganese porphine labeled antibody formed by an antigen-antibody reaction is incubated at pH 12.7 with an excess amount of luminol and the light produced by luminol is integrated for 1 to 2 minutes after initiation of the reaction.

Another object of the present invention is to improve stability of immunoassay components. This is done in two ways. The need for an unstable high energy oxidant or oxidant precursor is eliminated. Also, the method of the present invention allows replacement of an enzyme generally used with a more stable metal porphine. In the preferred example, a single detection reaction solution is prepared in advance and can be stored for a long period of time at room temperature before assays.

Another object of the present invention is to decrease non-specific (accidental) binding of the labeled catalyst to the solid phase used in immunoassay systems (e.g., ELISA). In many assays, sensitivity is limited by such non-specific binding and anything which limits non-specific binding directly results in improved sensitivity. This improvement of sensitivity is made possible by the replacement of an enzyme catalyst that has a large molecular weight (40,000-200,000 molecular weight) and is readily bound to the solid phase with a small porphine catalyst (1,000 molecular weight) that has less chance of sticking to the solid phase.

Another object of the present invention is to decrease complexity and cost of the instrument used for the assay. This is achieved by providing a chemical reaction that does not require the mixing of two or more separate solutions prior to use and that generates a long-lived chemiluminescence light signal. Many of the prior art procedures require the mixing of two or more unstable solutions immediately prior to the detection step. Sometimes this mixing has to be done in the optical cell of a photo detector. The present invention obviates this need by producing a light signal that persists longer than 15 minutes. By creating a long-lived light signal, the present invention allows the taking of a light measurement at various time periods after initiation of the chemiluminescence reaction.

Yet another object of the present invention is to increase the amount of light produced by a chemiluminescence reaction. This is advantageous because it allows more simple light detection devices to be used and shortens the time required to obtain data. This increase in the amount of light is made possible by the inclusion of certain additives in the chemiluminescence reaction solutions to enhance the chemiluminescence production of light more than 100 fold.

Still another object of the present invention is to provide methods which allows convenient performance of two simultaneous assays. This is made possible by the compatibility of the metal-porphine chemiluminescence chemistry with photosensitive dye chemiluminescence chemistry which requires light excitation. Metal porphine catalyzed chemiluminescence can be measured in the presence of the photosensitive dye and the photosensitive dye can be measured in the presence of metal porphine. This allows use of two separately labeled antibodies in the same assay step and separate detection of each. Simultaneous assays can thus be formulated for savings in assay time, labor and sample usage.

BRIEF EXPLANATION OF DRAWINGS

Fig. 1 is a graph illustrating effect of increasing Mn-sulfonatophenyl-4-porphine concentration on light output.

Fig. 2 is a graph illustrating enhancement of chemiluminescence catalytic action of Mn-porphine with Tween-20.

Fig. 3 is a graph illustrating an ELISA standard curve obtained using the conjugate of anti-endothelin antibody and Mn-mesotetra-4-carboxyphenyl porphine.

Fig. 4 is a graph illustrating a standard curve obtained using the conjugate of $_a$-fetoprotein and Mn-mesotetra-4-carboxyphenyl porphine.

Fig. 5 is a graph illustrating a standard curve for methylene blue dye catalyst in the presence of Mn-mesotetra-4-carboxyphenyl porphine.

Fig. 6 is a graph illustrating a standard curve for Mn-mesotetra-4-carboxyphenyl porphine in the presence of methylene blue dye catalyst.

SUMMARY OF THE INVENTION

According to the present invention, there are provided:
(1) A specific binding assay for measuring the presence of an analyte in a sample by detection of a metal porphine catalyst and binding member conjugate through at least one binding reaction which comprises carrying out the detection by:
    a. reacting the metal porphine catalyst with a chemilumigenic agent at high pH in the absence of any added oxidizing agent, and
    b. measuring chemiluminescence proportional to the concentration of said conjugate;
(2) A specific binding assay for measuring the presence of an analyte in a sample by detection of a metal porphine catalyst and binding member conjugate through at least one binding reaction which comprises carrying out the detection by:
    a. reacting the metal porphine catalyst with a chemilumigenic substance at high pH in the absence of any added oxidizing agent,
    b. enhancing the reaction of the metal porphine catalyst by an amphipathic substance, and
    c. measuring chemiluminescence proportional to the concentration of said conjugate;
(3) A specific binding assay for two analytes in a sample which comprises carrying out the detection by
    a. reacting (i) a photosensitive dye catalyst and binding member conjugate and (ii) a metal porphine catalyst and binding member conjugate in the same reaction mixture with a chemilumigenic substance at high pH in the absence of any added oxidizing agent,
    b. measuring chemiluminescence emitted from the metal porphine catalyst in the absence of light proportional to the concentration of said conjugate (ii), and
    c. measuring chemiluminescence from the photosensitive dye produced during or after light stimulation proportional to the concentration of said conjugate (i);
(4) A specific binding assay kit comprising:
    a. a first container containing a metal porphine catalyst and binding member conjugate, and
    b. a second container containing a chemilumigenic agent at a high pH with no additional oxidizing agent; and
(5) A specific binding assay kit comprising:
    a. a first container containing a metal porphine catalyst and binding member conjugate and
    b. a second container containing a chemilumigenic substance and an amphipathic substance at high pH with no additional oxidizing agent.

DETAILED DESCRIPTION OF THE INVENTION

4

The catalysts to be used in the present invention are, for example, phenyl porphines having a metal atom in the central nucleus. Iron and manganese are preferred as the metal atom and manganese is particularly preferred because catalysts made with manganese are more active than those made with iron. Other metals such as aluminum, zinc or lead are less preferred because catalysts made with them work very weakly or not at all. The phenyl rings in the phenyl porphines are preferably substituted at the para position with electron withdrawing residues such as carboxyl group or sulfone group. For coupling to a binding member such as antibody or DNA, it is preferred to use a carboxyl substituted metal porphine because there are many chemical procedures which can join the carboxyl group to other groups in the binding member, protein or nucleic acid. For coupling to protein such as avidin or antibody, it is most preferred to make an active derivative such as succinimide because succinimide can allow convenient coupling of the porphine catalyst to protein amino groups.

The catalyst, when attached to another molecule which is a binding member in this way, is herein referred to as a "conjugate". During the detection step, the conjugate can be quantitated by incubating it with chemilumiphore used as a chemilumigenic substance in a buffer solution (aqueous solution). The chemilumiphore is a molecule that generates light upon interaction with catalyst at basic pH. Luminol is the most preferred chemilumiphore but other chemilumiphores can be used such as luminol derivatives (e.g., isoluminol), lucigenin, benzopyrene and the like. Of course, chemilumigenic substances other than these chemilumiphores can also be used. Luminol is used at a concentration of at least 0.002 mM and preferably between 0.01 mM and 2 mM. For best detection sensitivity, the luminol is recrystallized from an alkaline solution and is stored in a plastic container. The buffer composition for maintaining pH constant is not particularly critical and a simple aqueous solution of sodium hydroxide is preferred. In the reaction of the metal porphine catalyst with the chemilumigenic substance, the pH is above pH 11, preferably between pH 12 and pH 13.5. The temperature of the reaction is also not critical and a temperature of between 15°C and 35°C is preferred.

For applications in which high light output is desired, it is preferred to use an enhancer obtained by dilution of an amphipathic substance. The following detergents, for example, are preferred additives to the detection solution to increase light output: Tween-20, Tween-40, Tween-60, Tween-80 and Tween-85. Tween-20 is most preferred because the enhancement by it is highest. Other detergents such as sorbitan monolaurate, sorbitan monooleate and Triton X-100 have utility although use of these leads to lower enhancement of light levels. When detergent enhancement is used, the optimum reaction conditions may be slightly altered. The preferred luminol concentration is higher in the presence of detergent and a concentration of between 0.2 and 2mM is particularly preferred.

Other enhancers include, for example, organic acids. The organic acids which have more than about 10 carbon atoms are desired and, particularly, unsaturated fatty acids are advantageously used. Among them, linoleic acid and linolenic acid are effective. They are used in a concentration from about 0.01% to about 1%, preferably from 0.05% to 0.2%.

The conditions suitable for metal porphine catalyzed chemiluminescence are also suitable for photosensitive dye catalyzed chemiluminescence. In the photosensitive dye system, a dye such as methylene blue becomes excited upon absorption of a light photon and makes singlet oxygen from dissolved molecular oxygen. Singlet oxygen reacts with luminol to generate light. Additionally, the excited dye may react directly with luminol to generate light.

In order to perform simultaneous measurements of metal porphine and photosensitive dye, it is preferred to use the same conditions specified above for metal porphine in the absence of the enhancer and to use a photosensitive dye having a lowest detectable concentration that produces light intensity equal to or greater than 5% of the highest light intensity produced by metal porphine in the assay. Because there is no chemiluminescence from photosensitive dye in the absence of light, metal porphine chemiluminescence detection is compatible with high concentrations of photosensitive dye.

The measurement and quantitation of chemiluminescence according to the method of the present invention can be carried out by a known method.

According to the quantitation method of the present invention, various substances, for example, those of medical, environmental and industrial importance and appear in liquid medium at very low concentrations can be determined. Particularly, the method of the present invention is suitable for quantitation of substances utilized in diagnostic assays. Examples thereof include proteins and hormones contained in body fluids such as human immunoglobulin G, human immunoglobulin M, human immunoglobulin A, human immunoglobulin E, human albumin, human fibrinogen (fibrin and its degradation products), $a$-fetoprotein, C-reactive protein, $b_2$-microglobulin, myoglobin, carcinoembryonic antigen, hepatitis virus antigen, human chorionic gonadotropin, human placental lactogen, insulin and the like as well as drugs.

The body fluids described above includes, for example, blood such as whole blood, serum, plasma, etc.

urine, bone marrow fluid, tissue extracts and the like.

For obtaining a reagent for the measurement, metal porphines such as carboxyl substituted manganese porphine can be chemically bound to hapten, antigen, antibody against them, RNA or DNA to prepare a metal porphine label.

As an embodiment of the assay kit utilizing the metal porphine label, a kit for a sandwich method is set forth below:

(1) an antibody immobilized on a support;

(2) the metal porphine conjugated to a second antibody;

(3) a standard sample of an analyte molecule;

(4) a buffer solution used for dilution of the above agents (2) and (3) and a sample to be tested (The buffer is not specifically limited in so far as it can be used for dilution of the agents and the sample. Examples thereof include phosphate buffer and glycine buffer of pH 6 to 9.);

(5) a buffer solution used for washing the support after incubation (The buffer is not specifically limited in so far as it can be used for washing the support. Examples thereof include phosphate buffer and glycine buffer.); and

(6) an agent necessary for the measurement of the metal porphine activity (Examples thereof include luminol which is dissolved in an alkaline solvent.).

The above kit can be used as follows:

A solution of a standard sample or a sample to be tested (about 10 to 200 )l) is diluted with the agent (4) and the diluted solution is reacted with the agent (1) at about 0 to 40°C. After the reaction for about 10 minutes to 24 hours, the mixture is washed with the agent (5) and the catalytic activity of the metal porphine bound to the support is measured. That is, the agent (6) (about 10 to 3000 )l) is added to the mixture to measure the amount of chemiluminescence in the reaction solution with a chemiluminescence assay apparatus for a certain period of time.

The assay kit of the present invention can be prepared in the form of powders, granules and liquids in which the above conjugate and chemilumigenic substance are contained in separate containers. The chemilumigenic substance can be optionally mixed with the amphipathic substance in a certain concentration. Also, buffers can be combined with them. The container is not specifically limited and includes those made of glass, plastic or metal. Each agent can be prepared according to a known method such as dissolution, dispersion, mixing, drying, granulation, lyophilization and the like.

The following Examples further illustrate the present invention in detail but are not to be construed to limit the scope thereof.

Example 1

Standard curve of Mn-mesotetra-4-sulfonatophenyl porphine

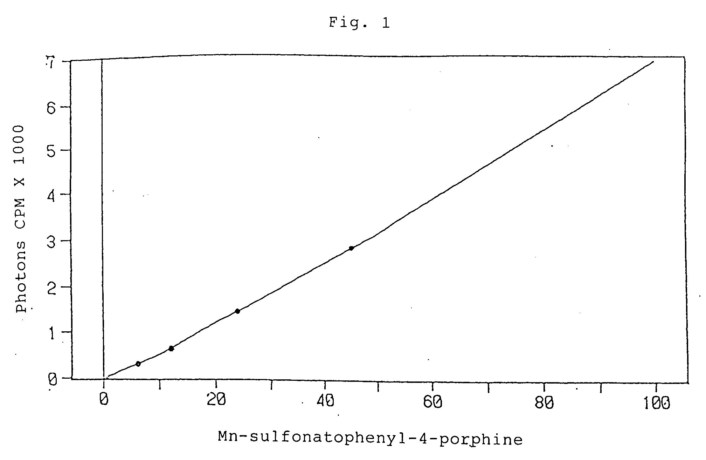

Mn-mesotetra-4-sulfonatophenyl porphine (Porphyrin Products, Logan, Utah, U.S.A.) was dissolved in 1mM NaOH and diluted to various concentrations in 0.01 mM luminol and 0.2 M NaOH solution. One ml samples were prepared in test tubes and placed into a photon counting luminometer. Light photons were counted from 50 seconds to 60 seconds after insertion into the luminometer. Results are shown in Fig. 1. The detection limit from this experiment (95% probability of distinguishing a lowest concentration from the zero level) was 0.9 pg of Mn-mesotetra-4-sulfonatophenyl porphine.

Example 2

Enhancement of chemiluminescence action of Mn-mesotetra-4-sulfonatophenyl porphine by Tween-20

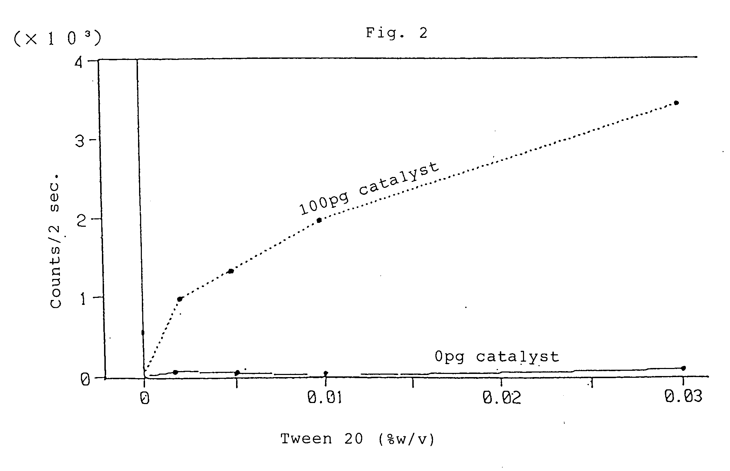

Mn-mesotetra-4-sulfonatophenyl porphine was dissolved in 1 mM NaOH. The resulting solution was diluted to 100 pg/ml and to 0 pg/ml with 1 ml solutions of 0.1 M NaOH and 1 mM luminol containing 0%, 0.0025%, 0.005%, 0.01% or 0.03% Tween-20 (Sigma Chemical Company, St. Louis, Mo., U.S.A.). One ml samples were prepared in test tubes and placed into a photon counting luminometer. Light photons were counted between 60 to 62 seconds after insertion of each sample into the luminometer. The results are shown in Fig. 2.

Example 3

Comparison of chemiluminescence action of various porphyrin derivatives

Mn-mesotetra-4-sulfonatophenyl porphine, Fe-mesotetra-4-sulfonatophenyl porphine, Mn-mesotetra-4-carboxyphenyl porphine, mesotetra-4-carboxyphenyl porphine, mesotetra-4-sulfonatophenyl porphine, Mn-protoporphyrin, Zn-protoporphyrin, Sn-protoporphyrin, Al-phthalocyanine tetrasulfonate and Chlorin E6 (Porphyrin Products, Logan, Utah, U.S.A.) as well as microperoxidase, Cu chlorphyllin and hematin (Sigma Chemical Company, St. Louis, Mo., U.S.A.) were tested. Each compound was diluted to 100 pg/ml and 1 ng/ml with a solution containing 0.5 mM luminol, 0.1 M NaOH and 0.01% Tween-20. Light was measured from 60 seconds to 62 seconds after insertion of each sample into a photon counting chemiluminometer. Table 1 summarizes results from this experiment. Results are expressed as a ratio of light emission catalyzed by each compound compared to that from Mn-mesotetra-4-sulfonatophenyl porphine.

Table 1

| Comparison of catalytic action of chemiluminescence of various porphyrin derivatives | |
|---|---|
| Compound tested | Relative activity |
| Mn-mesotetra-4-sulfonatophenyl porphine | 1 |
| Fe-mesotetra-4-sulfonatophenyl porphine | 0.53 |
| Mn-mesotetra-4-carboxyphenyl porphine | 0.77 |
| Mesotetra-4-carboxyphenyl porphine | 0 |
| Mesotetra-4-sulfonatophenyl porphine | 0 |
| Mn-protoporphyrin | 0.23 |
| Zn-protoporphyrin | 0 |
| Sn-protoporphyrin | 0 |
| Al-phthalocyanine tetrasulfonate | 0 |
| Chlorin E6 | 0 |
| Microperoxidase (containing heme) | 0 |
| Cu chlorphyllin | 0 |
| Hematin (containing heme) | 0.06 |

Example 4

Use of Mn-mesotetra-4-carboxyphenyl porphine in immunoassay for endothelin

A 0.1 M carbonate buffer solution (0.1 ml, pH 9.5) containing 50 )g/ml monoclonal anti-endothelin antibody was added to each well of white plastic microtiter plate (DYNATECH MicroFLUOR, Dynatech Laboratories, Inc. Chantilly, Va., U.S.A.) and was incubated for 3 hours at room temperature. The antibody preparation is described by Suzuki et al. [J. Immun. Methods, 118, 245-250 (1989)]. The wells were rinsed three times with phosphate buffered saline and then 0.3 ml of 25% Block Ace (Snow Brand Products, Sapporo, Japan) in phosphate buffered saline was added to each well. The plates thus prepared were stored at 6°C until use.

Endothelin-1 (Peptide Institute Inc., Osaka, Japan) was diluted into 1% BSA with a final 100 fold dilution into assay buffer (10% block ace, 0.4 M NaCl, 1 mM disodium ethylene diamine tetraacetic acid, 20 mM sodium phosphate, pH 7.0). Then, 0.2 ml portions (0pg/ml, 20pg/ml, 50pg/ml, 100pg/ml and 200pg/ml in replicates of three) of endothelin-containing assay buffer were added to each well of the microtiter plate prepared above and incubated for 16 hours. Each well was washed 3 times with phosphate buffered saline. Conjugate incubation buffer [20 mM sodium phosphate buffer (pH 7.0) containing 10% Block Ace, 0.5% BSA, 0.4 M NaCl, 1 mM disodium ethylene diamine tetraacetic acid] containing a 1:25 fold dilution of polyclonal anti-endothelin antibody Mn-mesotetra-4-carboxyphenyl porphine conjugate (100 )l) was added to each well.

The preparation procedure of said conjugate is as follows.

N-Hydroxysuccinimide (5 )mol) in dry dimethylformamide (DMF, 100 )l) was mixed with dicyclohexyl carbodiimide (7.5 )mol) in dry DMF and reacted at 4°C overnight in the dark. Anti-endothelin antibody (5 mg) in 0.05 M phosphate buffer (1 ml, pH 8) was mixed with the succinimide ester solution (40 )l) prepared above and reacted at 4°C overnight in the dark. The conjugate was separated by passage through a 1.5 x 45 cm column of Ultragel ACA34 resin equilibrated in 0.1 M sodium phosphate (pH 6.5). The plate was incubated at 4°C for 16 hours.

After reaction, the microtiter plate was washed three times with cold phosphate buffered saline and the activity of the antibody conjugate reacted with endothelin was measured.

For the antibody conjugate detection step, 100 )l solution containing 1.0 mM luminol (purified from an alkaline solution), 0.07 M NaOH, 0.03 M glycine and 0.1% Tween-20 was added to each well of the microtiter plate. Light emission from each well was measured with an ML1000 microtiter plate chemiluminometer (Dynatech, Inc., Chantilly, Va., U.S.A.).

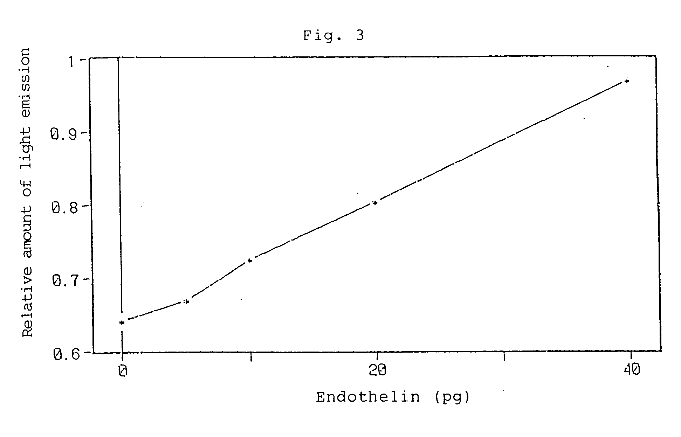

Fig. 3 shows an endothelin standard curve made by this method. There was a linear increase in light produced in the detection step with increasing endothelin concentration.

## Example 5

### Use of Mn-mesotetra-4-carboxyphenyl porphine in ELISA for $_a$-fetoprotein (AFP)

White microtiter plate wells were prepared with a anti-AFP antibody (type A-44 provided by Tokyo Standard Serum, Tokyo, Japan) according to the same manner as that described Example 4. AFP (Scripps Laboratories, Torrey Pines, Ca., U.S.A.) was diluted into 25% Block Ace, PBS and a final 100 fold dilution was made into incubation buffer (10% Block Ace, 0.5% BSA, 0.35% sodium alginate, PBS) just before use. To each well of the plate was added 195 )l of incubation buffer containing 0, 5, 12.5, 37.5 or 125 ng/ml AFP. Then, 5 )l of 2 )g/ml Mn-mesotetra-4-carboxyphenyl antibody conjugate was added to each well.

The conjugate preparation is as follows.

Mn-mesotetra-4-carboxyphenyl porphine (3 mg) was dissolved in DMF (2.1 ml). To this was added N-hydroxysuccinimide (4 mg) in 25 mM phosphate buffer (1.05 ml, pH 6.0) and water soluble carbodiimide (6.3 mg) in 25 mM phosphate buffer (1.05 ml, pH 6.0). After 1 hour of incubation at room temperature, anti-AFP (A295, Tokyo Standard Serum, Tokyo, Japan) in 25 mM phosphate buffer (4.2 ml, pH 6.0) was added. This was incubated overnight at 6°C. The derivatized protein was then separated from low molecular weight compounds by Sephadex G-50 gel filtration in the presence of 0.1 M phosphate buffer (pH 6.8).

After incubation for 45 minutes with shaking at 6°C, the plate was washed twice with PBS. A solution of 0.1 M NaOH, 1 mM luminol and 0.1% linolenic acid was added (100 )l per well) and the chemiluminescence measured with a microtiter plate reader according to the same manner as that described in Example 4.

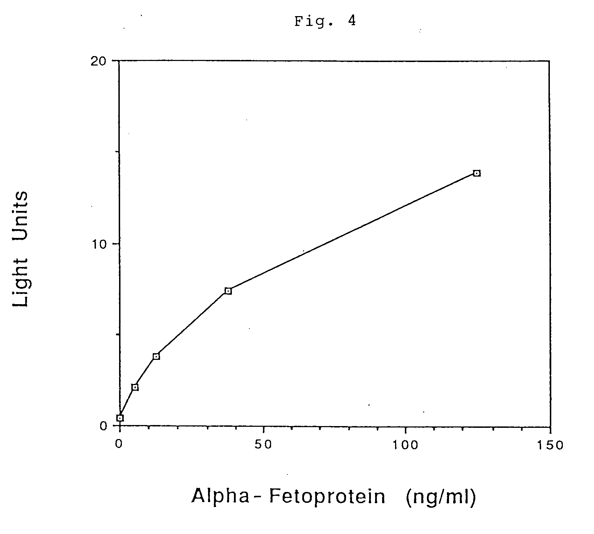

Fig. 4 shows an AFP standard curve made by this method. The detection limit (amount of analyte that produces a signal equal to twice the average background level) was calculated to be 1.5 ng/ml. Plasma concentrations of up to 15% could be used with this method and a typical adult plasma was found to contain less than 10 ng/ml AFP.

## Example 6

### Chemiluminescence from two different catalysts in the same assay tube

In this example, two different chemiluminescence catalyst systems are present in the same assay tube and are measured separately. One is a photosensitive dye (methylene blue) which reacts with luminol to make chemiluminescence. The second catalyst is Mn-mesotetra-4-carboxyphenyl porphine.

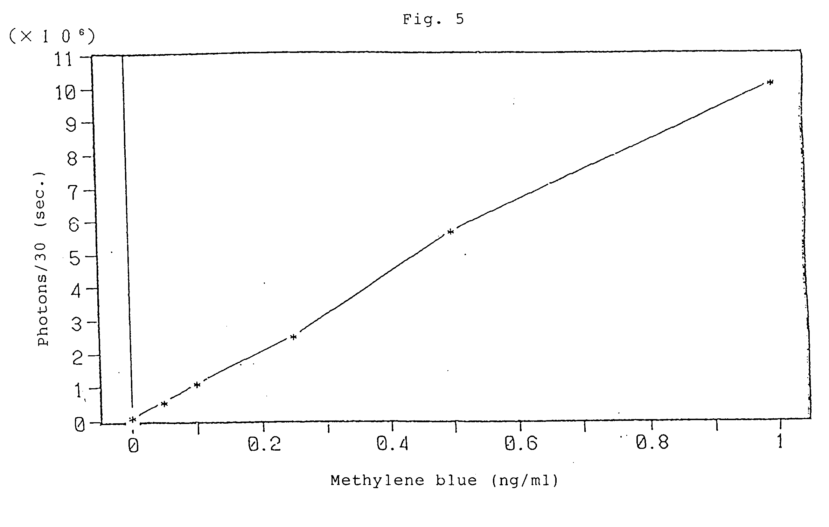

Both catalysts were dissolved in 20% ethanol and 80% water solution containing 1 mM luminol and 50 mM NaOH. Methylene blue was diluted to 1 ng/ml, 0.5 ng/ml, 0.25 ng/ml, 0.1 ng/ml and 0 ng/ml in the presence of 10 ng/ml Mn-mesotetra-4-carboxyphenyl porphine. The chemiluminescence was measured in a photon-counting chemiluminometer having a 5 mW 670 nm laser diode to excite the dye catalyst and a sharp 435nm cut-off filter to prevent 670 nm light from entering the photon multiplier tube. One ml solutions were prepared in 12 mm test tubes and photons were counted between 30 seconds and 60 seconds after insertion of each tube into the chemiluminometer. The background dark counts were subtracted from the measurements. The results are shown in Fig. 5.

In another experiment, Mn-mesotetra-4-carboxyphenyl porphine was diluted to concentrations of 10 ng/ml, 5 ng/ml, 2.5 ng/ml, 1 ng/ml, 0.5 ng/ml and 0 ng/ml with the same buffer containing 1 ng/ml methylene blue. Chemiluminescence was measured according to the same manner as described above except that the laser light was off and background was not subtracted.

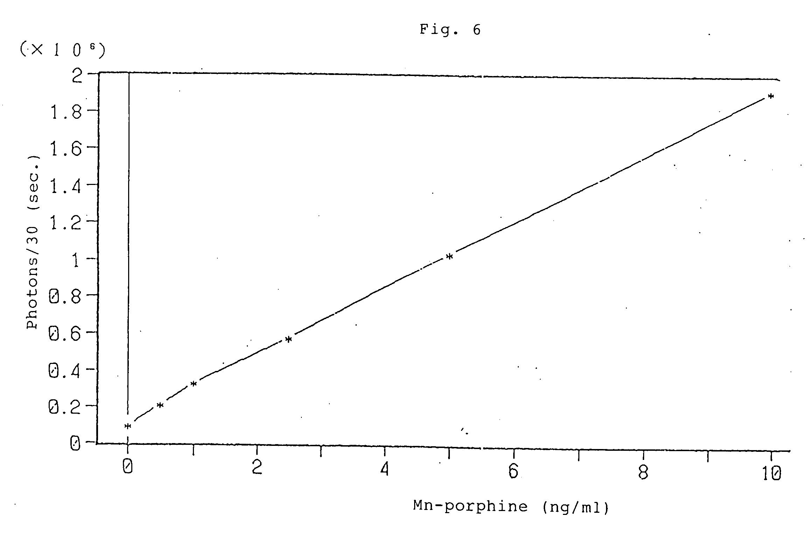

These results are shown in Fig. 6. Figs. 5 and 6 show that both catalysts can be independently quantitated from the same reaction mixture.

## Example 7

Enhancement of chemiluminescence with amphipathic substances

In this example, chemiluminescence is enhanced by addition of amphipathic substances to a solution of luminol and Mn porphine under alkaline conditions.

One milliliter portions of 0.5 mM luminol dissolved in 0.1 N NaOH aqueous solution were distributed into test tubes. Then, 10% aqueous solutions (10 )l) of compounds for chemiluminescence enhancement to be tested were added to the tubes and mixed, followed by measuring the amount of chemiluminescence for 1 minute (B). Further, 100ng/ml solutions (10 )l) of Mn-mesotetra-4-sulfonatophenyl porphine dissolved in aqueous 10 mM NaOH solutions were added and mixed, followed by measuring the amount of chemiluminescence for 1 minute (S). As a control, distilled water was used instead of an aqueous solution of a test compound.

Intensity of chemiluminescence enhancement activity of the test compounds was calculated according to the following formula:

chemiluminescence enhancement ratio = (S-B) test compound/(S-B) control

The results of this experiment are shown in Table 2.

Table 2

| Comparison of chemiluminescence enhancement activity | |
| --- | --- |
| Test compound | Chemiluminescence enhancement ratio |
| Distilled water (control) | 1.0 |
| Sodium lauryl sulfate | 2.9 |
| Linolenic acid | 175 |
| Linoleic acid | 159 |
| Hexamethylenediamine | 5.7 |
| Sodium deoxycholate | 1.8 |
| Tween-20 | 218 |
| Tween-40 | 272 |
| Tween-60 | 327 |
| Tween-80 | 114 |
| Tween-85 | 136 |
| Span-80 | 49 |
| Sodium octanate | 2.0 |
| Triton X-100 | 79 |
| Triton X-15 | 4.0 |
| Telgitol 4 | 2.8 |

As described hereinabove, according to the present invention, a specific binding assay can be carried out by simple reactions with high sensitivity, which can obviate inconvenience caused by using conventional unstable agents and save assay time, labor and reagent usage.

**Claims**

1. A specific binding assay for measuring the presence of an analyte in a sample by detection of a metal porphine catalyst and binding member conjugate through at least one binding reaction which comprises carrying out the detection by:
   a. reacting the said conjugate with a chemilumigenic agent at high pH without added oxidizing agent, and
   b. measuring chemiluminescence proportional to the concentration of said conjugate.

2. A specific binding assay according to claim 1, wherein the pH of step a is above pH 11.

3. A specific binding assay according to claim 1, wherein the metal porphine catalyst is meso-tetra substituted with 4-carboxyphenyl or 4-sulfonatophenyl residues.

9

4. A specific binding assay according to claim 1, wherein the porphine contains iron or manganese.

5. A specific binding assay according to claim 1, wherein the chemilumigenic agent is luminol or a luminol derivative.

6. A specific binding assay for measuring the presence of an analyte in a sample by detection of a metal porphine catalyst and binding member conjugate through at least one binding reaction which comprises carrying out the detection by:
   a. reacting the metal porphine catalyst with a chemilumigenic substance at high pH without added oxidizing agent,
   b. enhancing the reaction of the metal porphine catalyst by an amphipathic substance, and
   c. measuring chemiluminescence proportional to the concentration of said conjugate.

7. A specific binding assay according to claim 6, wherein the pH of step a is above pH 11.

8. A specific binding assay according to claim 6, wherein the metal porphine catalyst is meso-tetra substituted with 4-carboxyphenyl or 4-sulfonatophenyl residues.

9. A specific binding assay according to claim 6, wherein the porphine contains iron or manganese.

10. A specific binding assay according to claim 6, wherein the chemilumigenic agent is luminol or a luminol derivative.

11. A specific binding assay according to claim 6, wherein the amphipathic substance is a member selected from the group consisting of Tween 20, Tween 40, Tween 60 and Tween 80.

12. A specific binding assay for two analytes in a sample which comprises carrying out the detection by
   a. reacting (i) a photosensitive dye catalyst and binding member conjugate and (ii) a metal porphine catalyst and binding member conjugate in the same reaction mixture with a chemilumigenic substance at high pH without added oxidizing agent,
   b. measuring chemiluminescence emitted from the metal porphine catalyst in the absence of light proportional to the concentration of said conjugate (ii), and
   c. measuring chemiluminescence from the photosensitive dye produced during or after light stimulation proportional to the concentration of said conjugate (i).

13. A specific binding assay according to claim 12, wherein the pH of step a is above pH 11.

14. A specific binding assay according to claim 12, wherein the metal porphine catalyst is meso-tetra substituted with 4-carboxyphenyl or 4-sulfonatophenyl residues.

15. A specific binding assay according to claim 12, wherein the porphine contains iron or manganese.

16. A specific binding assay according to claim 12, wherein the chemilumigenic agent is luminol or a luminol derivative.

17. A specific binding assay kit comprising:
   a. a first container containing a metal porphine catalyst and binding member conjugate, and
   b. a second container containing a chemilumigenic agent at a high pH with no additional oxidizing agent.

18. A kit according to claim 17, wherein agent b is above pH 11.

19. A kit according to claim 17, wherein the metal porphine catalyst is meso-tetra substituted with 4-carboxyphenyl or 4-sulfonatophenyl residues.

20. A specific binding assay according to claim 17, wherein the porphine contains iron or manganese.

21. A specific binding assay according to claim 17, wherein the chemilumigenic agent is luminol or a luminol derivative.

22. A specific binding assay kit comprising:
    a. a first container containing a metal porphine catalyst and binding member conjugate and
    b. a second container containing a chemilumigenic substance and an amphipathic substance at a high pH without added oxidizing agent.

23. A kit according to claim 22, wherein the pH of agent b is above pH 11.

24. A kit according to claim 22, wherein the metal porphine catalyst is meso-tetra substituted with 4-carboxyphenyl or 4-sulfonatophenyl residues.

25. A kit according to claim 22, wherein the porphine contains iron or manganese.

26. A kit according to claim 22, wherein the chemilumigenic agent is luminol or a luminol derivative.

27. A kit according to claim 22, wherein the amphipathic substance is a member selected from the group consisting of Tween 20, Tween 40, Tween 60 and Tween 80.

Fig. 1

EP 0 480 361 A2

Fig. 2

Fig. 3

Relative amount of light emission

Endothelin (pg)

Fig. 4

Fig. 5

EP 0 480 361 A2

EP 0 480 361 A2

Fig. 6

$(\times 10^6)$

Photons/30 (sec.)

Mn-porphine (ng/ml)